Europäisches Patentamt

European Patent Office

Office européen des brevets

(11) Veröffentlichungsnummer: **0 315 875**
**A1**

# (12) EUROPÄISCHE PATENTANMELDUNG

(21) Anmeldenummer: 88118180.4

(22) Anmeldetag: 02.11.88

(51) Int. Cl.4: **A61K 9/50** , **A61K 9/52** , **B01J 13/02**

(30) Priorität: 11.11.87 DE 3738228

(43) Veröffentlichungstag der Anmeldung:
17.05.89 Patentblatt 89/20

(84) Benannte Vertragsstaaten:
AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Schmiedel, Rainer, Dr.
Gundelhardtstrasse 2
D-6233 Kelkheim (Taunus)(DE)
Erfinder: Sandow, Jürgen Kurt, Dr.
Am Haideplacken 22
D-6240 Königstein/Taunus(DE)

(54) **Verfahren zur Herstellung von bioabbaubaren Mikrokapseln wasserlöslicher Peptide und Proteine sowie nach diesem Verfahren erhaltene Mikrokapseln.**

(57) Es wird ein Verfahren zur Herstellung von biologisch abbaubaren Mikrokapseln wasserlöslicher Peptide/Proteine beschrieben. Der biologisch abbaubare Träger ist ein Gemisch aus PHB und PLGA. Die Kapseln werden durch Versprühen einer Suspension, Lösung oder Emulsion aus Wirk- und Trägerstoffen erhalten.

EP 0 315 875 A1

## Verfahren zur Herstellung von bioabbaubaren Mikrokapseln wasserlöslicher Peptide und Proteine sowie nach diesem Verf ahren erhaltene Mikrokapseln

Wasserlösliche Peptide und Proteine als Arzneistoffe werden wegen ihrer Zersetzung im sauren Milieu des Magens unter Umgehung des Gastrointestinaltraktes appliziert. In der Regel werden solche Peptide/Proteine parenteral als Injektionslösung oder nasal in Form einer Spraylösung angewendet.

Für eine Langzeitbehandlung bestimmter Krankheiten kann es erforderlich sein, den Wirkstoff als Depot-Arzneiform zu applizieren. Es wurde bereits in der Literatur beschrieben, daß Arzneistoffe, auch Peptide, in polymere Matrixsysteme inkorporiert werden und diese Matrices Z.B. in Form von Mikrokapseln oder stäbchenförmigen Implantaten hergestellt werden (vgl. z.B. EP-B-58481 und EP-B-52510).

Zur Herstellung der Matrix werden insbesondere bioabbaubare Polymere, die ein operatives Entfernen eines Polymerimplantates überflüssig machen, verwendet.

Die bioabbaubaren Polymere, die in der Literatur für diese Anwendung beschrieben werden, sind Polyamide, Polyanhydride, Polyester, Poly(ortho)ester und Polyacetale.

Die am häufigsten beschriebenen Polymeren sind die Polyester. Diese setzen sich aus Monomeren zusammen, die im menschlichen oder tierischen Körper vorkommen, wie z.B. Milchsäure, Glykolsäure oder 3-Hydroxybuttersäure.

Zur Herstellung von Mikrokapseln aus Polyestern werden in der Literatur verschiedene Verfahren beschrieben, zu denen neben der Phasenseparation ( = Ausfällen des Polymeren auf die Wirkstoffteilchenoberfläche), das solvent evaporation-Verfahren ( = Verdampfen des Polymerlösungsmittels aus einer Emulsion des Lösungsmittels in Wasser, bei dem das gelöste Polymer ebenfalls Wirkstoffteilchen, die sich in der Emulsion befinden, umhüllt) und auch die Sprühtrocknung gehören.

Bei wasserlöslichen Peptiden/Proteinen, die nach dem Phasenseparationsverfahren oder dem solvent evaporation-Verfahren mikroverkapselt werden können, stellt sich das Problem, daß sich das Peptid/Protein während des Verkapselungsvorganges durch Lösen im Wasser teilweise oder ganz der Verkapselung durch das Polymer entzieht.

Die Sprühtrocknung von Feststoffen aus ihrer Lösung kann unter geeigneten Herstellbedingungen in Gegenwart eines polymeren Hüllmaterials zur Ausbildung von Mikrokapseln führen. Während die Peptide/Proteine in der Regel gut wasserlöslich sind, lassen sich die bioabbaubaren Polyester in Wasser nicht lösen. Um nun die wasserlöslichen Peptide in Mikrokapseln zu überführen, kann man

a) den Wirkstoff mikronisiert in der organischen Polyesterlösung dispergieren

b) eine wäßrige Wirkstofflösung in der organischen Polymerlösung emulgieren

c) Wirkstoff und Polymer in einem Lösungsmittel oder einem Lösungsmittelgemisch lösen

und anschließend der Sprühtrocknung unterziehen.

Je nach angewandtem Verfahren resultieren, wie entsprechende Untersuchungen zeigen, unterschiedliche Wirkstofffreigabeprofile. Diesen Profilen gemeinsam aber ist, daß die Wirkstofffreigabe mit der Zeit in etwa exponentiell abnimmt und dadurch bedingt, um einen therapeutischen Effekt zu erzielen, zu gering ist.

Diese Aussage gilt insbesondere für die gut versprühbaren Homopolymeren Poly-(L)-lactid, Poly(DL)-lactid und Polyhydroxybuttersäure.

Es ist auch schon beschrieben worden, daß aus biologisch abbaubaren Copolymeren wie Poly-(DL)-lactid-co-glykolid ( = PLGA) Peptide über einen längeren Zeitraum in ausreichender Menge freigesetzt werden. Solche bioabbaubaren Copolymere lassen sich jedoch durch Sprühtrocknung nicht zu Mikrokapseln verarbeiten.

Überraschend wurde nun gefunden, daß sich Mischungen aus einem an sich gut versprühbaren Homopolymer mit einem an sich nicht versprühbaren Copolymer (PLGA) bis zu einem Gewichtsverhältnis von 50 Gewichtsprozent problemlos versprühen lassen.

Die Erfindung betrifft daher ein Verfahren zur Herstellung von biologisch abbaubaren Mikrokapseln wasserlöslicher Peptide und Proteine, dadurch gekennzeichnet, daß man das Peptid oder Protein in der Lösung einer Mischung aus dem Homopolymeren Polyhydroxybuttersäure (PHB) und dem Copolymer Polylactid-co-glykolid (PLGA), wobei das Mischungsverhältnis von PHB zu PLGA zwischen 99 : 1 bis 20 : 80 Gewichtsprozent liegt, suspendiert oder löst, oder eine wäßrige Lösung des Peptids oder Proteins in der Lösung einer Mischung aus PHB und PLGA, wobei das Mischungsverhältnis von PHB zu PLGA zwischen 99 : 1 bis 20 : 80 Gewichtsprozent liegt, emulgiert und die Suspension, Lösung oder Emulsion anschließend versprüht, sowie nach diesem Verfahren erhältliche Mikrokapseln.

Die Peptide/Proteine liegen zweckmäßigerweise in feinverteilter oder mikronisierter Form vor. Die Mikronisierung erfolgt z.B. in der Luftstrahlmühle; der Teilchendurchmesser beträgt zweckmäßigerweise < 20 µm. Im Polymergemisch ist der PHB-Anteil vorzugsweise im Bereich von 90 bis 40 Gew.-%, der PLGA-Anteil liegt insbesondere im Bereich von 10 bis 60 Gew.-%.

Der biologisch abbaubare Träger ist eine Mischung aus PHB und PLGA. Die Polyhydroxybuttersäure hat ein Molekulargewicht von 5000 bis 50000, vorzugsweise von 7000 bis 35000. Als PLGA kommt ein Copolymer aus Polylactid und Polyglykolid in Frage, im Bereich von 99 : 1 bis 1 : 99. Vorzugsweise sollte der Polylactid-Anteil im Bereich von 80 bis 50 Gew.-%, der Polyglykolid-Anteil sinngemäß im Bereich von 20 bis 50 Gew.-% liegen. Das Molekulargewicht des PLGA wird durch die Bestimmung der inhärenten Viskosität indirekt bestimmt. Die inhärente Viskosität, gemessen bei einer Polymerkonzentration von 1 mg/ml in Chlorform bei 25°C liegt im Bereich von 0,3 bis 0,8 dL/g, vorzugsweise 0,4 bis 0,6 dL/g.

Je nachdem welches Lösungsmittel eingesetzt wird, erhält man eine Suspension oder Lösung des Wirkstoffes in der Polymerlösung. Eine Suspension wird z.B. bei Verwendung von Chloroform oder Dichlormethan erhalten; eine Lösung kann durch Verwendung eines Lösungsmittelgemisches, wie z.B. Wasser/Ethanol/CHCl$_3$ (ternäres Gemisch) erzielt werden. Ebenso kann zur Erzeugung einer Lösung auch ein Lösungsmittel wie DMF eingesetzt werden.

Um eine Emulsion herzustellen, wird die wäßrige Peptid-/Proteinlösung in der Lösung des Polymergemisches in z.B. CHCl$_3$ oder CH$_2$Cl$_2$ emulgiert.

Je nach eingesetzem Peptid/Protein ist entweder das Suspensions-/Lösungsverfahren oder das Emulsionsverfahren besser geeignet. Die Versprühung der Suspension, Lösung oder Emulsionen oder Dispersionen erfolgt nach bekannten Methoden z.B. im Mini Spray Dryer der Firma Büchi.

Je nach angewandtem Polymerlösungsmittel (Dimethylformamid, Dichlormethan, Chloroform, z.T. in wäßriger Emulsion) werden beim Versprühen Temperaturen von 20°C bis 110°C, vorzugsweise von 45°C bis 95°C, benötigt, denen die Mikrokapseln nur für wenige Sekunden ausgesetzt sind. Damit ist das Verfahren sehr schonend.

Die Oberfläche der Mikrokapseln ist glatt; sie weist nur vereinzelt sichtbare Poren auf.

Im Querschnitt erscheinen die Mikrokapseln teilweise als Hohlkugeln mit einer 1 bis 3 µm dicken Wand, die je nach Herstellweise mehr oder weniger mit kleinen Hohlräumen erfüllt ist. Zum Teil werden aber auch gefüllte Kugeln gefunden.

Die Mikrokapseln fallen als feinteiliges Pulver an. Die sehr feinteiligen Pulver zeigen im rasterelektronenmikrospopischen Bild das Vorhandensein von nahezu kugelförmigen Partikeln mit einem Durchmesser $\leq$ 50 µm, die z.T. zu Agglomeraten vereinigt sind, die meist maximal 100 µm groß sind. Durch Variation der Verfahrensbedingungen kann man die Größe der Mikrokapseln variieren; der Durchmesser liegt bei 50 % der Kapseln meist zwischen 10 und 75 µm.

Die Beladung der Mikrokapseln mit Wirkstoff (= Wirkstoffgehalt) beträgt vorzugsweise 3 bis 17 %.

Als Wirkstoff eignen sich alle wasserlöslichen Peptide und Proteine und deren physiologisch verträgliche Salze. Die Peptide und Proteine können natürlichen, halbsynthetischen und synthetischen Ursprungs sein. Insbesondere kommen Buserelin, Protirelin, Somatoliberin und Angiogenin in Betracht.

Aus den Mikrokapseln wird der Wirkstoff verzögert abgegeben. Die Freigabe kann durch Zugabe von Freigaberegulatoren beeinflußt werden. Freigabebeschleuniger, wie z.B. Citronensäure, Lactose oder Glycin können erheblich zu einer steileren Freigabe beitragen. Die Freigabebeschleunigung kann sich negativ auf die Länge der Freigabedauer auswirken.

Als Freigabebeschleuniger kommen hydrophile Stoffe anorganischer, organischer oder polymerer Natur in Frage, z.B.

1. anorganische Stoffe: z.B. Natriumchlorid, Natriumdihydrogenphosphat, Siliciumdioxid (®Aerosil 200).

2. organische Stoffe: z.B. Citronensäure, Lactose, Mannit, Glycerin, DMSO, Essigsäure, Sojalecithin

3. polymere Stoffe: Polyethylenglykol, Humanalbumin, Polyoxyethylen-polyoxypropylencopolymer (z.B. ®Pluronic).

Die Freigabe wird insbesondere beeinflußt durch die Zusammensetzung des Polymerengemisches, die Wirkstoffbeladung und die Größe der Mikrokapseln. Mit Zunahme der Wirkstoffbeladung steigt der Initialrelease an, was gleichzeitig nach einigen Tagen zu niedrigen Freigaberaten führt, insbesondere wenn z.B. beim Buserelin die Mikrokapseln nach dem "Suspensionsverfahren" hergestellt wurden. Beim "Emulsionsverfahren" ist bei gleicher Wirkstoffkonzentration entweder der Initialrelease kleiner oder die täglichen Freigaberaten über 4 Wochen höher, so daß hier dieses Verfahren vorzuziehen ist.

Bei geringerer Wirkstoffbeladung wird die Wirkstofffreigabe durch Zunahme des PLGA-Gehaltes weniger beeinflußt als bei höherem Wirkstoffgehalt. Bei einem PHB/PLGA-Verhältnis von 70/30 verläuft z.B. bei einem Wirkstoffgehalt von 6,3 % die Freigabe noch relativ flach. Sie steigt bei 8,4 % initial deutlich an und verläuft über die 2. bis 4. Woche auf erhöhtem Niveau gegenüber 6,3 % (vgl. auch Beispiel 1).

**Beispiel 1**

a) 0,252 g Buserelinacetat werden in 3,0 g Wasser gelöst. Die wäßrige Lösung wird mit Hilfe eines Rotor-Stator-Homogenisators in einer wäßrigen Lösung von 2,63 g PHB 30.000 und 1,12 g PLGA 75:25 (PHB : PLGA = 70 : 30) in 92,0 g Chloroform emulgiert und in einem Laborsprühtrockner versprüht. Die Sprühparameter werden wie folgt eingestellt:

| 1. Eingangstemperatur | 90 °C |
|---|---|
| 2. Sprühflow | 500 Skalenteile |
| 3. Aspirator | 16 " |
| 4. Pumpenleistung | 3 " |

Die Mikrokapseln fallen als weißes, rieselfähiges Pulver an. Ausbeute: 1,4 g $\hat{=}$ 35 % der Theorie, Wirkstoffgehalt: 6,3 %.

b) In gleicher Weise werden Mikrokapseln hergestellt, unter Verwendung von 0,336 g Peptid und 2,56 g PHB 30000 und 1,10 g PLGA. 75 : 25 (PHB : PLGA = 70 : 30). Wirkstoffgehalt: 8,4 %. Ausbeute: 1,8 $\hat{=}$ 45 % der Theorie.

Für 20 mg Mikrokapseln wurden die in vitro-Freigaberaten wie folgt bestimmt: 20 mg Mikrokapseln werden in 2,0 ml einer Phosphatpufferlösung pH 7,4 eingebracht und 24 h lang bei +37 °C eluiert. Nach Trennung des Eluats von den Mikrokapseln wird das Eluat mit Hilfe eines Radioimmunoassays (RIA) auf den Buserelingehalt geprüft. Die zurückbleibenden Mikrokapseln werden erneut in 2,0 ml Phosphatpufferlösung gegeben und wiederum 24 h lang bei 37 °C eluiert. Dieser Prozeß wird über 28 Tage analog fortgesetzt.

Die aus den Eluaten bestimmten Buserelinacetat-Mengen sind in der folgenden Tabelle ausschnittsweise zusammengestellt:

|  | Wirkstoffgehalt 6,3 % | Wirkstoffgehalt 8,4 % |
|---|---|---|
| Tag 2 | 85 μg | 484 μg |
| Tag 7 | 31 μg | 51 μg |
| Tag 14 | 8 μg | 35 μg |
| Tag 21 | 5 μg | 19 μg |
| Tag 28 | 4 μg | 15 μg |

Vergleicht man die in vitro-Freigabeprofile der nach Sprühtrocknung herstellbaren Buserelin-Mikrokapseln auf Basis PHB (gemäß Beispiel 2a) mit dem nach Sprühtrocknung gewonnenen Mikrokapseln auf Basis PHB/PLGA-Mischung (gemäß Beispiel 2b), so ist das Freigabeprofil der 8,4 % beladenen Mikrokapseln auf Basis PHB 30.000 deutlich flacher verlaufend, als das der ebenfalls 8,4 % beladenen Mikrokapseln auf Basis PHB 30.000/PLGA 75 : 25 Mischung im Verhältnis 70 : 30 (vgl. Beispiel 2).

**Beispiel 2**

a) 0,336 g Busserelin werden in 4,0 g Wasser gelöst. Die wäßige Lösung wird mit Hilfe eines Rotor-Stator-Homogenisators in einer Lösung von 3,66 g PHB 30000 in 92,0 g Chloroform emulgiert und in einem Laborsprühtrockner versprüht. Die Sprühparameter entsprechen denen von Beispiel 1. Ausbeute: 2,4 g $\hat{=}$ 60 % der Theorie. Wirkstoffgehalt: 8,4 %.

b) Gleiche Darstellung wie Beispiel 1 b)

Die in vitro-Freigaberaten wurden analog Beispiel 1 bestimmt.

| | Wirkstoffgehalt 8,4 % | 8,4 % |
|---|---|---|
| | PHB 30000 | PHB 30000/PLGA 70/30 |
| Tag 2 | 318 $\mu$g | 484 $\mu$g |
| Tag 7 | 30 $\mu$g | 51 $\mu$g |
| Tag 14 | 6 $\mu$g | 35 $\mu$g |
| Tag 21 | 3 $\mu$g | 19 $\mu$g |
| Tag 28 | 3 $\mu$g | 15 $\mu$g |

## Beispiel 3

Unter Verwendung eines Polymerengemisches aus 1,88 g PHB 30000 und 1,88 g PLGA 75 : 25 (PHB : PLGA = 50 : 50) werden wie in Beispiel 1a) beschrieben Mikrokapseln mit einem Wirkstoffgehalt von 6,3 % hergestellt. Diese Mikrokapseln mit einem Wirkstoffgehalt von 6,3 % weisen nach einer vierwöchigen in vitro-Freigabeprüfung noch > 50 % des Wirkstoffgehaltes auf. Eine in etwa lineare Wirkstofffreigabe erfolgt über 3 Monate (Prüfungsmethode s. Beispiel 1)

| Wirkstoffgehalt 6,3 %, PHB 30000/PLGA 75: 25 50/50 | | | |
|---|---|---|---|
| | freigesetzte Menge | | freigesetzte Menge |
| Tag 2 | 84 $\mu$g | Tag 63 | 5 $\mu$g |
| Tag 7 | 31 $\mu$g | Tag 70 | 6 $\mu$g |
| Tag 14 | 7 $\mu$g | Tag 77 | 5 $\mu$g |
| Tag 21 | 5 $\mu$g | Tag 84 | 7 $\mu$g |
| Tag 28 | 4 $\mu$g | | |
| Tag 35 | 5 $\mu$g | | |
| Tag 42 | 7 $\mu$g | | |
| Tag 49 | 7 $\mu$g | | |
| Tag 56 | 5 $\mu$g | | |

## Ansprüche

1. Verfahren zur Herstellung von biologisch abbaubaren Mikrokapseln wasserlöslicher Peptide und Proteine, dadurch gekennzeichnet, daß man das Peptid oder Protein in der Lösung einer Mischung aus dem Homopolymeren Polyhydroxybuttersäure (PHB) und dem Copolymer Polylactid-co-glykolid (PLGA), wobei das Mischungsverhältnis von PHB zu PLGA zwischen 99 : 1 bis 20 : 80 Gewichtsprozent liegt, suspendiert oder löst, oder eine wäßrige Lösung des Peptids oder Proteins in der Lösung einer Mischung aus PHB und PLGA wobei das Mischungsverhältnis von PHB zu PLGA zwischen 99 : 1 bis 20 : 80 Gewichtsprozent liegt, emulgiert und die Suspension, Lösung oder Emulsion anschließend versprüht.

2. Verfahren gemäß Anspruch 1, dadurch gekennzeichnet, daß man eine Mischung mit einem PHB-Anteil von 90 bis 40 Gew.-% und einem PLGA-Anteil von 10 bis 60 Gew.-% verwendet.

3. Verfahren gemäß Anspruch 1, dadurch gekennzeichnet, daß man als Wirkstoff Buserelin, Protirelin, Somatoliberin oder Angiogenin oder ein physiologisch verträgliches Salz dieser Verbindungen verwendet.

4. Verfahren gemäß Anspruch 1, dadurch gekennzeichnet, daß man Buserelin oder ein physiologisch verträgliches Buserelin-Salz verwendet.

5. Verfahren gemäß Anspruch 1, dadurch gekennzeichnet, daß man zusätzlich einen Freigaberegulator verwendet.

6. Mikrokapseln erhältlich nach Verfahren gemäß Anspruch 1.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 190 833 (TAKEDA CHEMICAL IND.) * Seite 8, Zeilen 33-35; Seite 9, Zeilen 1-35; Seite 23, Zeilen 1-3 * --- | 1,6 | A 61 K 9/50 A 61 K 9/52 B 01 J 13/02 |
| A | FR-A-2 070 153 (E.I. DU PONT DE NEMOURS) * Seite 3, Zeile 26; Seite 12, Zeilen 2-33; Seite 17, Zeilen 14-34 * --- | 1,6 | |
| P,A | EP-A-0 251 631 (M.E. ANDERSON) * Spalte 4, Zeilen 14-30; Spalte 7, Zeilen 5-8; Spalte 11, Zeilen 1-10; Spalte 12, Zeilen 1-10 * --- | 1,6 | |
| A | EP-A-0 172 422 (HOECHST AG) * Seite 2, Zeilen 10-20; Seite 3, Zeilen 1-3 * --- | 1,3,4,6 | |
| A,D | EP-A-0 052 510 (SYNTEX) * Seite 13, Zeilen 3-35; Seite 23, Zeilen 1-8 * ----- | 1,6 | RECHERCHIERTE SACHGEBIETE (Int. Cl.4) A 61 K |

Der vorliegende Recherchenbericht wurde für alle Patentansprüche erstellt

| Recherchenort | Abschlußdatum der Recherche | Prüfer |
|---|---|---|
| DEN HAAG | 24-01-1989 | KERRES P.M.G. |